(19) Europäisches Patentamt
European Patent Office
Office européen des brevets

(11) Veröffentlichungsnummer: **0 102 324**
**A2**

(12) # EUROPÄISCHE PATENTANMELDUNG

(21) Anmeldenummer: 83810338.0

(22) Anmeldetag: 25.07.83

(51) Int. Cl.³: A 61 K 9/50

(30) Priorität: 29.07.82 CH 4597/82

(43) Veröffentlichungstag der Anmeldung: 07.03.84 Patentblatt 84/10

(84) Benannte Vertragsstaaten: AT BE CH DE FR GB IT LI LU NL SE

(71) Anmelder: CIBA-GEIGY AG
Postfach
CH-4002 Basel(CH)

(72) Erfinder: Hauser, Helmut, Dr.
Schwarzbachstrasse 91
CH-8713 Uerikon(CH)

(54) **Lipide und Tenside in wässriger Phase.**

(57) Die vorliegende Erfindung betrifft ein neues, vorteilhaftes Verfahren zur Herstellung von unilamellaren Liposomen in wässriger Phase, indem man eine homogene Mischung eines ionischen Tensids und eines Lipids dispergiert. Die Bildung der unilamellaren Liposome erfolgt spontan, d.h. ohne zusätzliche äussere Energiezufuhr. Die verfahrensgemäss erhältlichen Liposome können als Träger von Wirkstoffen unterschiedlichster Art therapeutisch verwendet werden.

EP 0 102 324 A2

Croydon Printing Company Ltd

CIBA-GEIGY AG
Basel (Schweiz)

4-14035/+

## Lipide und Tenside in wässriger Phase

Gegenstand der vorliegenden Erfindung ist ein Verfahren zur Herstellung von unilamellaren Liposomen in wässriger Phase.

Liposomen sind in der Literatur in zahlreichen Veröffentlichungen beschrieben worden. Ihr Aufbau und ihre Verwendung ist Gegenstand vieler Untersuchungen. Man unterscheidet unilamelare Liposomen mit einer Doppelschicht aus Lipiden von multilamellaren Liposomen mit mehreren Doppelschichten aus Lipiden, die zwiebelförmig angeordnet sind.

Unilamellare Liposomen haben einen Durchmesser von ca. 200 bis 50000 Å, vorzugsweise ca. 200 bis 30000 Å. Die kugelförmige Hülle besteht aus einer Doppelschicht der Lipidkomponenten, z.B. amphipatischen Lipiden, z.B. Phospholipiden, z.B. Phosphatidsäure, Lecithin oder Kephalin, und gegebenenfalls neutralen Lipiden, z.B. Cholesterin. Diese Doppelschicht umschliesst einen Innenraum, der eine wässrige Phase enthält.

Es besteht grosses Interesse an der therapeutischen Verwendung von Liposomen als Träger von Wirkstoffen unterschiedlichster Art. So sind Liposomen als Träger von Proteinen, z.B. Antikörpern oder Enzymen, Hormonen, Vitaminen oder Genen oder zu analytischen Zwecken als Träger von markierten Verbindungen vorgeschlagen worden. Als

Beispiel sei die US-Patentschrift 3,993,754 genannt, welche ein chemotherapeutisches Verfahren bei der Behandlung von Tumorzellen unter Verwendung von Liposomen als Träger zum Gegenstand hat.

Der betreffende Wirkstoff wird entweder bei der Bildung der Liposomen oder nachträglich durch Diffusion verkapselt. Die Herstellung von Liposomen und die Verkapselung des Wirkstoffs kann auf verschiedene Weise erfolgen und ist in dem Uebersichtsartikel von Kaye, St.B., Cancer Treatment Reviews (1981) 8, 27-50 beschrieben. Weitere Verfahren zur Herstellung von Liposomen zwecks Verkapselung von Wirkstoffen sind ebenfalls durch Barenholz et al, in Biochemistry, Vol. 16, No. 12, 2806-2810, sowie in den Deutschen Offenlegungsschriften (DOS) 28 19 855, 29 02 672, 25 32 319 und 28 42 608, in der US-Patentschrift 4,053,585 und in der Europäischen Patentanmeldung 36 676 beschrieben.

Nach den bisher bekannt gewordenen Verfahren löst man beispielsweise die Lipidkomponenten, z.B. Phospholipide, z.B. Phosphatidsäure, Lecithin oder Kephalin, und gegebenenfalls neutrale Lipide, z.B. Cholesterin, in einem organischen Lösungsmittel, z.B. Chloroform oder Benzol, auf. Nach dem Eindampfen bleibt eine homogene Schicht, z.B. eine Filmschicht, der betreffenden Lipidkomponenten zurück. Man dispergiert anschliessend die Lipidkomponenten in einer wässrigen Phase, welche den betreffenden Wirkstoff enthält, z.B. durch Schütteln. Bei der anschliessenden Behandlung mit Ultraschall bilden sich unilamellare Liposomen, welche den Wirkstoff verkapseln.

Nach dem Verfahren der vorliegenden Erfindung lassen sich auf einfache Weise ohne apparativen Aufwand wässrige Phasen herstellen, welche kleine unilamellare Liposomen (KUL) mit einem Durchmesser von ca. 200-600 Å und grosse unilamellare Liposomen (GUL) mit einem Durchmesser von ca. 600-3000 Å enthalten. Mittels geeigneter Trennmethoden, z.B. Gelfiltration oder einer Ultrafiltrationszelle, kann man kleine von grossen unilamellaren Liposomen trennen.

Die vorliegende Erfindung hat ein Verfahren zur Herstellung von unilamellaren Liposomen zum Gegenstand, dadurch gekennzeichnet, dass man eine homogene Mischung eines ionischen Tensids und eines Lipids in wässriger Phase bei einer Konzentration niedriger als die kritische Mizellbildungskonzentration (cmc-critical micelle concentration) des Tensids in der betreffenden Phase dispergiert und, wenn notwendig, die erhältliche wässrige Phase neutralisiert und, wenn erwünscht, die erhältlichen unilamellaren Liposomen anreichert und/oder abtrennt.

Die weiter vorn und im folgenden genannten allgemeinen Begriffe haben im Rahmen der Beschreibung der vorliegenden Erfindung vorzugsweise die folgenden Bedeutungen:

Der im Zusammenhang mit organischen Resten, z.B. Niederalkyl, Niederalkylen, Niederalkoxy, Niederalkanoyl etc., verwendete Ausdruck "Nieder" bedeutet, dass solche organische Reste, falls nicht ausdrücklich anders definiert, bis zu 7 und bevorzugt bis zu 4 Kohlenstoffatome enthalten.

Die Herstellung der homogenen Mischung eines ionischen Tensids und eines Lipids erfolgt in an sich bekannter Weise und ist in dem Abschnitt "Herstellung der homogenen Schicht der Lipidkomponenten" beschrieben.

Ein ionisches Tensid ist ein kationisches oder anionisches Tensid.

Ein kationisches Tensid ist beispielsweise eine Verbindung der Formel

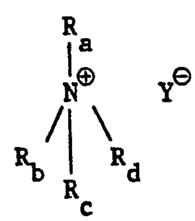

(I A),

worin $R_a$ einen gegebenenfalls substituierten Kohlenwasserstoffrest, $R_b$ Niederalkyl, Phenylniederalkyl oder Hydroxy, $R_c$ und $R_d$ Niederalkyl oder $R_b$ und $R_c$ zusammen mit dem Stickstoffatom einen gegebenenfalls an einem Kohlenstoffatom substituierten, aliphatischen Heterocyclus und $R_d$ Niederalkyl oder $R_b$, $R_c$ und $R_d$ zusammen mit dem Stickstoffatom einen gegebenenfalls an einem Kohlenstoffatom substituierten, aromatischen Heterocyclus und $Y^{\ominus}$ ein Anion darstellen.

In einem kationischen Tensid der Formel (IA) ist ein gegebenenfalls substituierter, aliphatischer Kohlenwasserstoffrest $R_a$ beispielsweise durch Aryloxyniederalkoxy substiutiertes Niederalkyl, geradkettiges oder verzweigtes Alkyl mit 7-22, insbesondere 12-20, Kohlenstoffatomen oder Alkenyl mit 8-20, insbesondere 12-20, Kohlenstoffatomen und 1-4 Doppelbindungen.

Aryl in Aryloxyniederalkoxy ist beispielsweise Phenyl, welches durch geradkettiges Niederalkyl mit 1-4 Kohlenstoffatomen, z.B. Methyl, Aethyl oder n-Propyl, oder durch verzweigtes Alkyl mit 3-10 Kohlenstoffatomen, z.B. Isobutyl, tert-Butyl, Amyl, Neopentyl, 2- oder 3-Methylpentyl, 2,2- oder 2,3-Dimethylbutyl, 2- oder 3-Methylhexyl, 3-Aethylpentyl, 2,2-, 2,3-, 2,4- oder 3,3-Dimethylpentyl, 4-Methylheptyl, 2,2,2- 2,2,4-, 2,3,3- oder 2,3,4-Trimethylpentyl, 1,1,3,3-Tetramethylbutyl oder 2,2,3,3-Tetramethylbutyl mono- oder disubstituiert sein kann.

Niederalkoxy in Aryloxyniederalkoxy ist beispielsweise Methoxy, Aethoxy, n-Propoxy oder n-Butoxy.

Niederalkyl $R_a$, welches durch Aryloxyniederalkoxy substituiert ist, ist beispielsweise Aryloxyniederalkoxymethyl oder 2-Aryloxyniederalkoxyäthyl, z.B. Aryloxymethoxymethyl, 2-Aryloxymethyloxyäthyl, 2-Aryloxyäthoxymethyl oder 2-(2-Aryloxyäthoxy)-äthyl, z.B. Phenoxymethoxymethyl, 2-Phenoxymethoxyäthyl, 2-Phenoxyäthoxymethyl, 2-(2-Phenoxyäthoxy)-äthyl, 2-, 3- oder 4-Methylphenoxymethoxymethyl, 2-(2-Methylphenoxymethoxy)-äthyl, 2-(3-Methylphenoxymethoxy)-äthyl,

2-(4-Methylphenoxymethoxy)-äthyl, 2-(2-Methylphenoxy)-äthoxymethyl, 2-(3-Methylphenoxy)-äthoxymethyl, 2-(4-Methylphenoxy)-äthoxymethyl, 2-[2-(2-Methylphenoxy)-äthoxy]-äthyl, 2-[2-(3-Methylphenoxy)-äthoxy]-äthyl, 2-[2-(4-Methylphenoxy)-äthoxy]-äthyl, 4-(1,1,3,3-Tetramethylbutyl)-phenoxymethoxymethyl, 2-[4-(1,1,3,3-Tetramethylbutyl)-phenoxymethoxy]-äthyl, 2-[4-(1,1,3,3-Tetramethylbutyl)-phenoxy]-äthoxymethyl, 2-[2-(4-(1,1,3,3-Tetramethylbutyl)-phenoxy)-äthoxy]-äthyl, 2-Methyl-4-(1,1,3,3-tetramethylbutyl)-phenoxymethoxymethyl, 2-[2-Methyl-4-(1,1,3,3-tetramethylbutyl)-phenoxymethoxy]-äthyl, 2-[3-Methyl-4-(1,1,3,3-tetramethylbutyl)-phenoxymethoxy]-äthyl, 2-[2-(2-Methyl-4-(1,1,3,3-tetramethylbutyl)-phenoxy)]-äthoxymethyl, 2-[2-(3-Methyl-4-(1,1,3,3-tetramethylbutyl)-phenoxy)]-äthoxymethyl, 2-[2-(2-Methyl-4-(1,1,3,3-tetramethylbutyl)-phenoxy)-äthoxy]-äthyl oder 2-[2-(3-Methyl-4-(1,1,3,3-tetramethylbutyl)-phenoxy)-äthoxy]-äthyl.

Niederalkyl $R_a$, welches durch Aryloxyniederalkoxy substituiert ist, ist vorzugsweise 2-[2-(2-Methyl-4-(1,1,3,3-tetramethylbutyl)-phenoxy)-äthoxy]-äthyl und 2-[2-(3-Methyl-4-(1,1,3,3-tetramethylbutyl)-phenoxy)-äthoxy]-äthyl.

Geradkettiges oder verzweigtes Alkyl $R_a$ mit 7-22, insbesondere 12-20, Kohlenstoffatomen, ist beispielsweise n-Heptyl, 2-Methylhexyl, 3-Methylhexyl, 3-Aethylpentyl, 2,2-, 2,3-, 2,4- oder 3,3-Dimethylpentyl, n-Octyl, 4-Methylheptyl, 2,2,3-, 2,2,4-, 2,3,3-, 2,3,4-Trimethylpentyl, n-Nonyl, n-Decyl, n-Undecyl, n-Dodecyl (Lauryl), n-Tridecyl, n-Tetradecyl (Myristyl), n-Pentadecyl, n-Hexadecyl (Cetyl), n-Heptadecyl, n-Octadecyl (Stearyl), n-Nonadecyl oder n-Eicosyl (Arachinyl).

Bevorzugt ist geradkettiges Alkyl mit einer geraden Anzahl von 12-20 Kohlenstoffatomen, beispielsweise n-Dodecyl (Lauryl), n-Tetradecyl (Myristyl), n-Hexadecyl (Cetyl), n-Octadecyl (Stearyl) oder n-Eicosyl (Arachinyl).

Alkenyl $R_a$ mit 8-20, insbesondere 12-20, Kohlenstoffatomen und 1-4 Dopppelbindungen ist beispielsweise 1-Octenyl, 1-Nonenyl, 1-Decenyl, 1-Undecenyl, 1-Dodecenyl, 9-cis-Dodecenyl (Lauroleyl), 1-Tridecenyl, 1-Tetradecenyl, 9-cis-Tetradecenyl (Myristoleyl), 1-Pentadecenyl, 1-Hexadecenyl, 9-cis-Hexadecenyl (Palmitoleinyl), 1-Heptadecenyl, 1-Octadecenyl, 6-cis-Octadecenyl (Petroselinyl), 6-trans-Octadecenyl (Petroselaidinyl), 9-cis-Octadecenyl (Oleyl), 9-trans-Octadecenyl (Elaidinyl), 9-cis-12-trans-Octadecadienyl (Linoleyl), 9-cis-11-trans-13-tans-Octadecatrienyl (α-Eläostearinyl), 9-trans-11-trans-13-trans-Octadecatrienyl (ß-Eläostearinyl), 9-cis-12-15-cis-Octadecatrienyl (Linolenyl), 9-, 11-, 13-, 15-Octadecatetraenyl (Parinaryl), 1-Nonadecenyl, 1-Eicosenyl, 9-cis-Eicosenyl (Gadoleinyl), 5-, 11-, 14-Eicosatrienyl oder 5-, 8-, 11-, 14-Eicosatetraenyl (Arachidonyl).

Bevorzugt ist Alkenyl mit 12-20 Kohlenstoffatomen und einer Doppelbindung, beispielsweise 9-cis-Dodecenyl (Lauroleyl), 9-cis-Tetradecenyl (Myristoleyl), 9-cis-Hexadecenyl (Palmitoleinyl), 6-cis-Octadecenyl (Petroselinyl), 6-trans-Octadecenyl (Petroselaidinyl), 9-cis-Octadecenyl (Oleyl), 9-trans-Octadecenyl (Elaidinyl) oder 9-cis-Eicosenyl (Gadoleinyl).

Niederalkyl $R_b$, $R_c$ oder $R_d$ ist beispielsweise Methyl oder Aethyl.

Phenylniederalkyl $R_b$ ist beispielsweise Benzyl oder 2-Phenyläthyl.

Ein aliphatischer Heterocyclus, welcher von $R_b$ und $R_c$ zusammen mit dem Stickstoffatom gebildet wird, ist beispielsweise ein monocyclischer, fünf- oder sechsgliedriger Aza-, Oxaaza- oder Thiazacyclylrest, z.B. Piperidino, Morpholino oder Thiamorpholinio.

Substituenten dieses Heterocylus sind die Substituenten $R_a$ und $R_d$ am Stickstoff sowie gegebenenfalls an einem Kohlenstoffatom Niederalkyl, z.B. Methyl, Aethyl, n-Propyl oder n-Butyl.

Ein Heterocyclus, welcher von $R_b$ und $R_c$ zusammen mit dem Stickstoffatom gebildet wird und an einem Kohlenstoffatom durch Niederalkyl substituiert ist, ist z.B. 2-, 3- oder 4-Methylpiperidinio, 2-, 3- oder 4-Aethylpiperidinio oder 2- oder 3-Methylmorpholinio.

Ein aromatischer Heterocyclus, welcher von $R_b$, $R_c$ und $R_d$ zusammen mit dem Stickstoffatom gebildet wird, ist beispielsweise ein monocyclischer, fünf- oder sechsgliedriger, Aza-, Diaza-, Oxaaza- oder Thiazacyclylrest, z.B. Pyridinio, Imidazolinio, Oxazolinio oder Thiazolinio oder beispielsweise ein benzokondensierter Monoazabicyclylrest, z.B. Chinolinio oder Isochinolinio.

Substituenten diese Heterocyclus sind der Rest $R_a$ am Stickstoffatom sowie gegebenenfalls an einem Kohlenstoffatom Niederalkyl, z.B. Methyl oder Aethyl, Hydroxyniederalkyl, z.B. Hydroxymethyl oder 2-Hydroxyäthyl, Oxo, Hydroxy oder Halogen, z.B. Chlor oder Brom.

Ein Heterocyclus, welcher von $R_b$, $R_c$ und $R_d$ zusammen gebildet wird und an einem Kohlenstoffatom durch die genannten Reste substituiert ist, ist beispielsweise 2- oder 4-Niederalkylpyridinio, z.B. 2- oder 4-Methyl oder 2- oder 4-Aethylpyridinio, Diniederalkylpyridinio, z.B. 2,6-Dimethyl-, 2-Methyl-3-äthyl-, 2-Methyl-4-äthyl-, 2-Methyl-5-äthyl-, oder 2-Methyl-6-äthylpyridinio, 2-, 3- oder 4-Halogenpyridinio, z.B. 2-, 3- oder 4-Chlorpyridinio oder 2-, 3- oder 4-Brompyridinio, 2-Niederalkylimidazolinio, -oxazolinio oder -thiazolinio, z.B. 2-Methyl- oder 2-Aethylimidazolinio, -oxazolinio oder -thiazolinio oder 2-Niederalkyl-8-halogenchinolinio, z.B. 2-Methyl-8-chlorchinolinio.

Ein Anion $Y^{\ominus}$ ist beispielsweise ein Halogenid-, z.B. Fluorid-, Chlorid- oder Bromid-, Niederalkanoat, z.B. Formiat- oder Acetat-, Hydrogensulfat-, Niederalkylsulfat-, z.B. Methyl- oder Aethylsulfat-, Niederalkylsulfonat-, z.B. Methyl-, oder Arylsulfonat-, z.B. Phenylsulfonat- oder Toluolsulfonation.

Ein Anion $Y^{\ominus}$ ist vorzugsweise ein Halogenid-, z.B. Chlorid- oder Bromidion.

Ein kationisches Tensid der Formel IA ist vorzugsweise N-Benzyl-N,N-dimethyl-N-2-[2-(4-(1,1,3,3-tetramethylbutyl)-phenoxy)-äthoxy]-äthylammoniochlorid, N-Benzyl-N,N-dimethyl-N-2-[2-(3-methyl-4-(1,1,3,3-tetramethylbutyl)-phenoxy)-äthoxy]-äthylammoniochlorid (Methylbenzethoniumchlorid), n-Dodecyltrimethylammoniochlorid oder -bromid, Trimethyl-n-tetradecylammoniochlorid oder -bromid, n-Hexadecyltrimethylammoniochlorid oder -bromid (Cetyltrimethylammoniumchlorid oder -bromid), Trimethyl-n-octadecylammoniochlorid oder -bromid, Aethyl-n-dodecyldimethylammoniochlorid oder -bromid, Aethyldimethyl-n-tetradecylammoniochlorid oder -bromid, Aethyl-n-hexadecyldimethylammoniochlorid oder -bromid, Aethyldimethyl-n-octadecylammoniochlorid oder -bromid, n-Alkyl-benzyldimethylammoniochlorid oder -bromid (Benzalkoniumchlorid oder -bromid), z.B. Benzyl-n-dodecyldimethylammoniochlorid oder bromid, Benzyldimethyl-n-tetradecylammoniochlorid oder -bromid, Benzyl-n-hexadecyldimethylammoniochlorid oder -bromid oder Benzyldimethyl-n-octadecylammoniochlorid oder -bromid, N-(n-Decyl)-pyridiniochlorid oder -bromid, N-(n-Dodecyl)-pyridiniochlorid oder -bromid, N-(n-Tetradeyl)-pyridiniochlorid oder -bromid, N-(n-Hexadecyl)-pyridiniochlorid oder -bromid (Cetylpyridiniumchlorid) oder N-(n-Octadecyl)- pyridiniochlorid oder -bromid oder eine Mischung von diesen Tensiden.

Ein anionisches Tensid ist beispielsweise

a) eine Verbindung der Formel:

$$[R_a-(O-A)_m-B]^{\ominus}Z^{\oplus} \qquad (I\ B)$$

worin $R_a$ einen gegebenenfalls substituierten Kohlenwasserstoffrest, A Niederalkylen, m null (direkte Bindung) oder eins, B die Sulfonat- oder Sulfatgruppe und $Z^{\oplus}$ ein einwertiges Kation darstellen, oder

b) eine Verbindung der Formel:

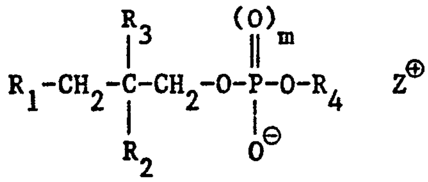 (I C),

worin m null oder eins ist, einer der Reste $R_1$ und $R_2$ Wasserstoff, Hydroxy, Niederalkyl mit 1-4 C-Atomen und der andere Rest Alkyl, Alkenyl, Alkoxy, Alkenyloxy oder Acyloxy mit je 10-20 C-Atomen, $R_3$ Wasserstoff oder Niederalkyl mit 1-4 C-Atomen und $R_4$ gegebenenfalls substituiertes Niederalkyl mit 1-7 C-Atomen, einen Kohlehydratrest mit 5-12 C-Atomen oder, wenn beide Reste $R_1$ und $R_2$ Wasserstoff oder Hydroxy bedeuten, einen Steroidrest bedeuten, und $Z^{\oplus}$ ein einwertiges Kation bedeutet, oder

c) eine Verbindung der Formel

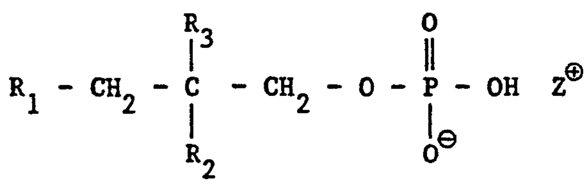 (I D),

worin $R_1$, $R_2$, $R_3$ und $Z^{\oplus}$ die unter Formel (I C) genannten Bedeutungen haben.

In einem anionischen Tensid der Formel (I B) hat der gegebenenfalls substituierte Kohlenwasserstoffrest $R_a$ die weiter vorn unter Formel I A genannten Bedeutungen und ist vorzugsweise geradkettiges oder verzweigtes Alkyl mit 7-22, insbesondere 12-20, Kohlenstoffatomen und Alkenyl mit 6-20, insbesondere 12-20, Kohlenstoffatomen und 1-4 Doppelbindungen.

In einem anionischen Tensid der Formel IB ist $R_a$ in erster Linie geradkettiges Alkyl mit einer geraden Anzahl von 12-20 Kohlenstoffatomen, beispielsweise n-Dodecyl (Lauryl), n-Tetradecyl (Myristyl), n-Hexadecyl (Cetyl), n-Octadecyl (Stearyl) oder

n-Eicosyl (Arachinyl), oder Alkenyl mit 12-20 Kohlenstoffatomen und 1 Doppelbindung, beispielsweise 9-cis-Dodecenyl (Lauroleyl)-, 9-cis-Tetradecenyl (Myristoleyl), 9-cis-Hexadecenyl (Palmitoleinyl)- 6-cis-Octadecenyl (Petroselinyl), 6-trans-Octadecenyl (Petroselaidinyl), 9-cis-Octadecenyl (Oleyl), 9-trans-Octadecenyl (Elaidinyl) oder 9-cis-Eicosenyl (Gadoleinyl).

A mit der Bedeutung Niederalkylen ist beispielsweise Methylen, Aethylen, n-Propylen oder n-Butylen.

Das Kation $Z^{\ominus}$ ist ein Alkalimetallkation, z.B. das Lithium-, Natrium- oder Kaliumion, oder ein Tetraniederalkylammoniumion, z.B. Tetramethylammonium.

Ein anionisches Tensid der Formel IB ist vorzugsweise ein Alkalimetallalkylsulfat (m = o), z.B. Natrium oder Kalium-n-dodecyl (lauryl)-sulfat, -n-tetradecyl (myristyl)-sulfat, -n-hexadecyl (cetyl)-sulfat oder -n-octadecyl (stearyl)-sulfat, ein Alkalimetallalkyläthersulfat (m = 1), z.B. Natrium- oder Kalium-n-dodecyloxyäthylsulfat, -n-tetradecyloxyäthylsulfat, -n-hexadecyloxyäthylsulfat oder -n-octadecyloxyäthylsulfat oder , ein Alkalimetallalkansulfonat, z.B. Natrium- oder Kalium-n-dodecansulfonat, -n-tetradecansulfonat, -n-hexadecansulfonat oder -n-octadecansulfonat.

In einem anionischen Tensid der Formel I C ist Niederalkyl $R_1$, $R_2$ oder $R_3$ mit 1-4 C-Atomen bevorzugt Methyl, ferner Aethyl, n-Propyl, oder n-Butyl.

Alkyl $R_1$ oder $R_2$ ist vorzugsweise n-Decyl,-n-Undecyl, n-Dodecyl (Lauryl), n-Tridecyl, n-Tetradecyl (Myristyl), n-Pentadecyl, n-Hexadecyl (Cetyl), n-Octadecyl (Stearyl) und n-Eicosyl (Arachinyl).

Alkenyl $R_1$ oder $R_2$ ist vorzugsweise 9-cis-Dodecenyl (Lauroleyl), 9-cis-Tetradecenyl (Myristoleyl), 9-cis-Hexadecenyl (Palmitoleinyl), 6-cis-Octadecenyl (Petroselinyl), 6-trans-Octadecenyl (Petroselaidinyl), 9-cis-Octadecenyl (Oleyl), 9-trans-Octadecenyl (Elaidinyl) und 9-cis-Eicosenyl (Gadoleinyl).

Alkoxy $R_1$ oder $R_2$ ist vorzugsweise n-Decyloxy, n-Dodecyloxy (Lauryloxy), n-Tetradecyloxy (Myristyloxy), n-Hexadecyloxy (Cetyloxy), n-Octadecyloxy (Stearyloxy) und n-Eicosyloxy (Arachinyloxy).

Alkenyloxy $R_1$ oder $R_2$ ist vorzugsweise 9-cis-Dodecenyloxy (Lauroleyloxy), 9-cis-Tetradecenyloxy (Myristoleyloxy), 9-cis-Hexadecanyloxy (Palmitoleinyloxy), 6-cis-Octadecenyloxy (Petroselinyloxy), 6-trans-Octadecenyloxy (Petroselaidinyloxy), 9-cis-Octadecenyloxy (Oleyloxy), 9-trans-Octadecenyloxy (Elaidinyloxy) und 9-cis-Eicosenyl (Gadoleinyloxy).

Acyloxy $R_1$ oder $R_2$ ist beispielsweise Alkanoyloxy oder Alkenoyloxy.

Alkanoyloxy $R_1$ oder $R_2$ ist vorzugsweise n-Decanoyloxy, n-Dodecanoyloxy (Lauroyloxy), n-Tetradecanoyloxy (Myristoyloxy), n-Hexadecanoyloxy, n-Hexadecanoyloxy (Palmitoyloxy), n-Octadecanoyloxy (Stearoyloxy) und n-Eicosoyloxy (Arachinoyloxy).

Alkenoyloxy $R_1$ oder $R_2$ ist vorzugsweise 9-cis-Dodecenyloxy (Lauroleoyloxy), 9-cis-Tetradecenoyloxy (Myristoleoyloxy), 9-cis-Hexadecenoyloxy (Palmitoleinoyloxy), 6-cis-Octadecenoyloxy (Peteroselinoyloxy), 6-trans-Octadecenoyloxy (Petroselaidinoyloxy), 9-cis-Octadecenoyloxy (Oleoyloxy), 9-trans-Octadecenoyloxy (Elaidinoyloxy) und 9-cis-Eicosenoyl (Gadoleinoyloxy).

Niederalkyl $R_4$ mit 1-7 C-Atomen ist z.B. Methyl, Aethyl, Isopropyl, n-Propyl, Isobutyl oder n-Butyl, und kann durch saure Gruppen, z.B. Carboxy oder Sulfo, saure und basische Gruppen, z.B. Carboxy und Amino, wobei die Aminogruppe sich in α-Stellung zur Carboxygruppe

befindet, freie oder verätherte Hydroxygruppen, wobei zwei verätherte Hydroxygruppen durch einen bivalenten Kohlenwasserstoffrest, z.B. durch Methylen, Aethylen, Aethyliden, 1,2-Propylen oder 2,2-Propylen, miteinander verbunden sein können, Halogen, z.B. Chlor oder Brom, Niederalkoxycarbonyl, z.B. Methoxy- oder Aethoxycarbonyl, oder durch Niederalkansulfonyl, z.B. Methansulfonyl, substituiert sein.

Substituiertes Niederalkyl $R_4$ mit 1-7 C-Atomen ist vorzugsweise Carboxyniederalkyl, z.B. Carboxymethyl, 2-Carboxyäthyl oder 3-Carboxy-n-propyl, ω-Amino-ω-carboxyniederalkyl, z.B. 2-Amino-2-carboxyäthyl oder 3-Amino-3-carboxy-n-propyl, Hydroxyniederalkyl, z.B. 2-Hydroxyäthyl oder 2,3-Dihydroxypropyl, Niederalkoxyniederalkyl, z.B. Methoxy- oder Aethoxymethyl, 2-Methoxyäthyl oder 3-Methoxy-n-propyl, Niederalkylendioxyniederalkyl, z.B. 2,3-Aethylendioxypropyl oder 2,3-(2,2-Propylen)-dioxypropyl, oder Halogenniederalkyl, z.B. Chlor oder Brommethyl, 2-Chlor- oder 2-Bromäthyl, 2- oder 3-Chlor- oder 2-oder 3-Brom-n-propyl.

Ein Kohlehydratrest $R_4$ mit 5-12 C-Atomen ist beispielsweise ein natürlicher Monosaccharidrest, der sich von einer als Aldose oder Ketose vorliegenden Pentose oder Hexose ableitet.

Eine als Aldose vorliegende Pentose ist z.B. D-Ribose, D-Arabinose, D-Xylose oder D-Lyxose.

Eine als Ketose vorliegende Pentose ist z.B. D-Ribulose oder D-Xylulose.

Eine als Aldose vorliegende Hexose ist z.B. D-Allose, D-Altrose, D-Glucose, D-Mannose, D-Galactose oder D-Talose.

Eine als Ketose vorliegende Hexose ist z.B. D-Psicose, D-Fructose, D-Sorbose oder D-Tagatose.

Eine Hexose liegt vorzugsweise in zyklischer Form vor, z.B. als Pyranose (Aldose), z.B. α- oder ß-D-Glucopyranose, oder als Furanose, z.B. α- oder ß-D-Fructose. Der Pyranosylrest ist vorzugsweise durch die in 1- oder 6-Stellung und der Furanosylrest durch in 1- oder 5-Stellung befindliche Hydroxygruppe mit der Phosphatidylgruppe (m = 1) verestert.

Ein Kohlehydratrest $R_4$ mit 5-12 C-Atomen ist ferner ein natürlicher Disaccharidrest, z.B. ein aus zwei Hexosen gebildeter Disaccaridrest, der sich beispielsweise durch Kondensation von zwei Aldosen, z.B. D-Glucose oder D-Galactose, oder einer Aldose, z.B. D-Glucose mit einer Ketose, z.B. Fructose, ableitet. Aus zwei Aldosen gebildete Disaccharide, z.B. Lactose oder Maltose, sind vorzugsweise über die in 6-Stellung des betreffenden Pyranosylrests befindliche Hydroxygruppe mit der Phosphatidylgruppe verestert. Aus einer Aldose und einer Ketose gebildete Disaccharide, z.B. Saccharose, sind vorzugsweise über die in 6-Stellung des Pyranosylrests oder über die in 1-Stellung des Furanosylrest befindliche Hydroxygruppe mit der Phosphatidylgruppe (m = 1) verestert.

Ein Kohlehydratrest $R_4$ mit 5-12 C-Atomen ist ferner ein derivatisierter Mono- oder Disaccharidrest, worin beispielsweise die Aldehydgruppe und/oder ein oder zwei endständige Hydroxygruppen zu Carboxygruppen oxydiert sind, z.B. ein D-Glucon-, D-Glucar- oder D-Glucoronsäurerest, welche vorzugsweise als zyklische Lactonreste vorliegen. Ebenso können in einem derivatisierten Mono- oder Disaccharidrest Aldehyd- oder Ketogruppen zu Hydroxygruppen reduziert sein, z.B. Inosit, Sorbit oder D-Mannit, oder Hydroxygruppen durch Wasserstoff, z.B. Desoxyzucker, z.B. 2-Desoxy-D-ribose, L-Rhamnose oder L-Fucose, oder durch Aminogruppen, z.B. Aminozucker, z.B. D-Glucosamin oder D-Galactosamin, ersetzt sein.

Ein Kohlehydrat $R_4$ kann ebenfalls ein durch Umsetzung eines der genannten Mono- oder Disaccharide mit einem starken Oxydationsmittel, z.B. Perjodsäure, gebildetes Spaltprodukt sein.

Ein Steroidrest $R_4$ ist beispielsweise ein Sterinrest, der über die in 3-Stellung des Steroidgerüsts befindliche Hydroxygruppe mit der Phosphatidylgruppe (m = 1) verestert ist.

Ein Sterinrest ist beispielsweise Lanosterin, Sitosterin, Koprostanol, Cholestanol, Glycocholsäure, Ergosterin oder Stigmasterin, vorzugsweise Cholesterin.

Wenn $R_4$ einen Steroidrest darstellt, sind $R_1$ und $R_2$ vorzugsweise Hydroxy und $R_3$ ist Wasserstoff.

$Z^{\oplus}$ hat die unter Formel I B genannten Bedeutungen und ist vorzugsweise Natrium oder Kalium.

In einen anionischen Tensid der Formel I C ist vorzugsweise m eins, $R_1$ Alkyl, z.B. n-Dodecyl (Lauryl), n-Tridecyl, N-Tetradecyl (Myristyl), n-Pentacedyl, n-Hexadecyl (Cetyl), n-Heptadecyl oder n-Octadecyl (Stearyl), Alkoxy, z.B. n-Dodecyloxy (Lauryloxy), n-Tetradecyloxy (Myristyloxy),n-Hexadecyloxy (Cetyloxy), oder n-Octadecyloxy (Stearyloxy), Acyloxy, z.B. Lauroyloxy, Myristoyloxy, Palmitoyloxy oder Stearoyloxy, $R_2$ Wasserstoff oder Hydroxy, $R_3$ Wasserstoff oder Niederalkyl, z.B. Methyl, $R_4$ Niederalkyl, z. B. Methyl oder Aethyl, Niederalkyl substituiert durch saure und basische Gruppen, z.B. Carboxy und Amino, z.B. ω-Amino-ω-carboxyniederalkyl, z.B. 2-Amino-2-carboxyäthyl oder 3-Amino-3-carboxy-n-propyl, Hydroxyniederalkyl, z.B. 2-Hydroxyäthyl oder 2,3-Hydroxypropyl, Niederalkylendioxyniederalkyl, z.B. 2,3-Aethylendioxypropyl oder 2,3-(2,2-Propylen)-dioxypropyl, Halogenniederalkyl, z.B. 2-Chlor- oder 2-Bromäthyl, einen Kohlenhydratrest mit 5-12 C-Atomen, z.B. Inosit, oder einen Steroidrest, z.B. ein Sterin, z.B. Cholesterin und $Z^{\oplus}$ Natrium oder Kalium.

Ein anionisches Tensid der Formel I C ist in erster Linie das Natrium- oder Kaliumsalz des Lysophosphatidylserins, z.B. das Natrium- oder Kaliumsalz des Lysophosphatidylserins aus dem Rinderhirn oder das Natrium- oder Kaliumsalz eines synthetischen Lysophosphatidylserins, z.B. Natrium- oder Kalium-1-myristoyl- oder -1-palmitoyllysophosphatidylserin, oder das Natrium- oder Kaliumsalz des Lysophosphatidylglycerins.

In einem anionischen Tensid der Formel I D haben $R_1$, $R_2$, $R_3$ und $Z^{\oplus}$ die unter Formel IC genannten Bedeutungen. Das Kation $Z^{\oplus}$ ist vorzugsweise Natrium oder Kalium. Das Wasserstoffatom an der Phosphatgruppe kann durch ein zweites Kation $Z^{\oplus}$ oder das Magnesiumion ersetzt sein.

In einem anionischen Tensid der Formel I D ist vorzugsweise $R_1$ Alkyl, z.B. n-Dodecyl (Lauryl), n-Tridecyl, n-Tetradecyl (Myristyl), n-Pentacedyl, n-Hexadecyl (Cetyl), n-Heptadecyl oder n-Octadecyl (Stearyl), Alkoxy, z.B. n-Dodecyloxy (Lauryloxy), n-Tetradecyloxy (Myristyloxy), n-Hexadecyloxy (Cetyloxy), oder n-Octadecyloxy (Stearyloxy), Acyloxy, z.B. Lauroyloxy, Myristoyloxy, Palmitoyloxy oder Stearoyloxy, $R_2$ Wasserstoff oder Hydroxy und $R_3$ Wasserstoff oder Niederalkyl, z.B. Methyl, und $Z^{\ominus}$ Natrium oder Kalium.

Ein anionisches Tensid der Formel I D ist in erster Linie das Natrium- oder Kaliumsalz einer natürlichen Phosphatidsäure, z.B. Ei-Phosphatidsäure, das Natrium- oder Kaliumsalz einer natürlichen Lysophosphatidsäure, z.B. Ei-Lysophosphatidsäure, das Natrium- oder Kaliumsalz einer synthetischen Lysophosphatidsäure, z.B. 1-Lauroyl-, 1-Myristoyl- oder 1-Palmitoyllysophosphatidsäure.

Ein Lipid, welches in der wässrigen Phase dispergiert wird, ist beispielsweise eine Verbindung der Formel

$$R_1 - CH_2 - \underset{\substack{| \\ R_2}}{\overset{\substack{R_3 \\ |}}{C}} - CH_2\,O - \underset{\substack{| \\ OH}}{\overset{\substack{(O)_m \\ \|}}{P}} - O - R_4 \qquad \text{(I C')},$$

worin m, $R_1$, $R_2$, $R_3$ und $R_4$ die unter Formel I C genannten Bedeutungen haben, $R_4$ ist ausserdem durch Triniederalkylammonio, z.B. Trimethylammonio, oder Amino substituiertes Niederalkyl, z.B. 2-Trimethylammonioäthyl (Cholinyl).

Ein Lipid ist vorzugsweise ein Lipid der Formel (I C') worin m eins, $R_1$ und $R_2$ Acyloxy, $R_3$ Wasserstoff und $R_4$ 2-Trimethylammonioäthyl oder 2-Aminoäthyl darstellen.

Ein solches Lipid ist z.B. ein natürliches Lecithin, z.B. Ei-Lecithin oder Lecithin aus Sojabohnen ($R_4$ = 2-Trimethylammonioäthyl), und ein natürliches Kephalin, z.B. Ei-Kephalin oder Kephalin aus Sojabohnen ($R_4$ = 2-Aminoäthyl).

Ausserdem sind als zusätzliche Lipide synthetische Lecithine ($R_4$ = 2-Trimethylammonioäthyl) und synthetische Kephaline ($R_4$ = 2-Aminoäthyl) der Formel (I C') bevorzugt, worin $R_1$ und $R_2$ identische Acyloxyreste, z.B. Lauroyloxy, Oleoyloxy, Linoyloxy, Linoleoyloxy oder Arachinoyloxy bedeuten, z.B. Dilauroyl-, Dimyristoyl-, Dipalmitoyl-, Distearoyl-, Diarachinoyl-, Dioleoyl-, Dilinoyl-, Dilinoleoyl-, oder Diarachinoyllecithin oder -kephalin, $R_1$ und $R_2$ verschiedene Acyloxyreste, z.B. $R_1$ Palmitoyloxy und $R_2$ Oleoyloxy, z.B. 1-Palmitoyl-2-oleoyl-lecithin oder -kephalin, $R_1$ und $R_2$ identische Alkoxyreste, z.B. Tetradecyloxy oder Hexadecyloxy, z.B. Ditetradecyl-oder Dihexadecyllecithin oder -kephalin, $R_1$ Alkenyl und $R_2$ Acyloxy, z.B. ein Plasmalogen ($R_4$ = Trimethylammonioäthyl), oder $R_1$ Acyloxy, z.B. Myristoyloxy oder Palmitoyloxy, und $R_2$ Hydroxy, z.B. natürliches oder synthetisches Lysolecithin oder Lysokephalin, z.B. 1-Myristoyl- oder 1-Palmitoyllysolecithin oder -kephalin, und $R_3$ Wasserstoff darstellen.

Ein geeignetes Lipid ist ferner ein Lipid der Formel I C', worin eins ist, $R_1$ einen Alkenylrest, $R_2$ einen Acylamidorest, $R_3$ Wasserstoff und $R_4$ 2-Trimethylammonioäthyl (Cholinrest) darstellen. Ein solches Lipid ist unter dem Namen Sphingomyelin bekannt.

Ein geeignetes Lipid ist ausserdem ein Lysolecithin-Analoges, z.B. 1-Lauroyl-1,3-propandiol-3-phosphorylcholin, ein Monoglycerid, z.B. Monoolein oder Monomyristin, ein Cerebrosid, ein Gangliosid oder ein Glycerid, welches keine freie oder veresterte Phosphoryl- oder Phosphonylgruppe in 3-Stellung enthält. Ein solches Glycerid ist beispielsweise ein Diacylglycerid oder 1-Alkenyl-1-hydroxy-2-acylglycerid mit den genannten Acyl- bzw. Alkenylgruppen, worin die 3-Hydroxygruppe durch einen der genannten Kohlenhydratreste, z.B. einen Galactosylrest, veräthert ist, z.B. ein Monogalactosylglycerin.

Ein geeignetes Lipid ist ferner ein neutrales Lipid, welches in Zellmembranen enthalten und nur in apolaren organischen Lösungsmitteln, z.B. in Chloroform, löslich ist. Neutrale Lipide sind beispielsweise Steroide, z.B. Oestradiol oder Sterine, z.B. Cholesterin, ß-Sitosterin, Desmosterin, 7-Keto-Cholesterin oder ß-Cholestanol, fettlösliche Vitamine, z.B. Vitamin A, z.B. Vitamin $A_1$ oder $A_2$, Vitamin E, Vitmin K, z.B. Vitamin $K_1$ oder $K_2$ oder Vitamin $D_2$ oder $D_3$.

Die homogene Mischung besteht vorzugsweise aus einem Tensid der Formel I A, insbesondere N,N-Dimethyl-N-2-[2-(4-(1,1,3,3-tetramethylbutyl)-phenoxy)-äthoxy]-äthylammoniochlorid, N-Benzyl-N,N-dimethyl-N-2-[2-(3-methyl-4-(1,1,3,3-tetramethylbutyl)-phenoxy)-äthoxy]-äthylammoniochlorid (Methylbenzethoniumchlorid), n-Dodecyltrimethylammoniochlorid oder -bromid, Trimethyl-n-tetradecylammoniochlorid oder-bromid, n-Hexadecyltrimethylammoniochlorid oder -bromid (Cetyltrimethylammoniumchlorid oder -bromid), Trimethyl-n-octadecylammoniochlorid oder -bromid, Aethyl-n-dodecyldimethylammoniochlorid oder -bromid, Aethyldimethyl-n-tetradecylammoniochlorid oder -bromid, Aethyl-n-hexadecyldimethylammoniochlorid oder -bromid, Aethyldimethyl-n-octadecylammoniochlorid oder -bromid, n-Alkylbenzyldimethylammoniochlorid oder -bromid (Benzalkoniumchlorid oder -bromid), z.B. Benzyl-n-dodecyldimethylammoniochlorid oder -bromid, Benzyldimethl-n-tetradecylammoniochlorid oder -bromid, Benzyl-n-hexadecyldimethylammoniochlorid oder -bromid oder Benzyldimethyl-n-octadecylammoniochlorid oder -bromid, N-(n-Decyl)-pyridiniochlorid oder -bromid, N-(n-Dodecyl)-pyridiniochlorid oder -bromid, N-(n-Tetradecyl)-pyridiniochlorid oder -bromid, N-(n-Hexadecyl)-pyridiniochlorid oder -bromid (Cetylpyridiniumchlorid) oder N-(n-Octadecyl)-pyridiniochlorid oder -bromid oder einem anionischen Tensid der Formel IB, insbesondere Natrium oder Kalium-n-dodecyl (lauryl)-sulfat, -n-tetradecyl (myristyl)-sulfat, -n-hexadecyl (cetyl)-sulfat oder -n-octadecyl (stearyl)-sulfat, Natrium- oder Kalium-n-dodecyloxyäthylsulfat, -n-tetradecyloxyäthylsulfat, -n-hexadecyloxyäthylsulfat oder -n-octadecyloxyäthylsulfat, oder einem anionischen Tensid der Formel I C, insbesondere Natrium- oder Kalium-2,2-dimethyl-3-palmitoyloxypropylhydrogenphosphat, Natrium- oder Kalium-1-palmitoyllysophosphatidylglycerin, Natrium- oder Kalium-1-palmitoyllysophosphatidylserin, und einem Lipid der Formel I C', worin $R_1$ und $R_2$ Acyloxy, z.B. Lauroyloxy, Myristoyloxy, Palmitoyloxy oder Stearoyloxy, $R_3$ Wasserstoff und $R_4$ 2-Trimethylammonioäthyl, z.B. ein natürliches Kephalin, z.B. Ei-kephalin-oder Kephalin aus Sojabohnen, oder 2-Aminoäthyl, z.B. ein natürliches Lecitin, z.B. Ei-Lecithin oder Lecithin aus Sojabohnen, bedeuten.

Die weiter vorn und im folgenden genannten Tenside und Lipide mit einem chiralen Kohlenstoffatom können auch als racemische Mischungen oder als optisch reine Enantiomere vorliegen.

In der homogenen Mischung beträgt das ungefähre Molverhältnis inonisches Tensid zu Lipid ca. 0,1 bis ca. 2 zu 1, bevorzugt ca. 0,8 bis ca. 1,2 zu 1.

Die homogene Mischung, z.B. den zuvor hergestellten Film oder Schaum, dispergiert man anschliessend in wässriger Phase, welche die zu verkapselnden Stoffe, z.B. Agrochemikalien, z.B. Schädlingsbekämpfungsmittel, Duftstoffe, Härtungsmittel, Farbstoffe oder pharmazeutischen Wirkstoffe, z.B. Peptide, z.B. Muramylpeptide, in gelöster, kolloider oder suspendierter Form Tenside enthält.

Man dispergiert beispielsweise durch Schütteln oder Rühren der wässrigen Phase, welche die zuvor hergestellte homogene Mischung enthält. Dabei findet die Bildung von unilamellaren Liposomen (KUL) und (GUL) spontan (spontaneous vesiculation), d.h. ohne zusätzliche Energiezufuhr von aussen und mit grosser Geschwindigkeit statt. Die Konzentration an Tensid, Lipid und Einschlussverbindung kann erhöht werden, bis die kritische Mizellbildungskonzentration (cmc) des betreffenden ionischen Tensids in der betreffenden wässrigen Phase erreicht ist.

Oberhalb der kritischen Mizellbildungskonzentration werden bevorzugt Mizellen geildet. Dieser Vorgang ist in vielen Fällen durch Verschwinden der Opaleszenz, z.B. Klarwerden der wässrigen Phase, erkennbar. Die cmc ist eine variable Richtgrösse für die Menge eines ionischen Tensids, welche man in einem bestimmten Volumen Wasser unter Vermeidung von Mizellbildung dispergieren kann. Auf den Zahlenwert der cmc hat die Struktur des hydrophoben Rests des Tensids Einfluss: Je grösser die Kettenlänge, desto niedriger der cmc-Wert. Raumbeanspruchende Substituenten im hydrophoben Rest, z.B. ein aromatischer Rest, setzen die cmc ebenfalls herab. Funktionelle Gruppen, z.B. Doppelbindungen, welche den hydrophoben Charakter des hydrophoben Restes abschwächen, erhöhen die cmc. Diese wird ferner von sämtlichen dispergierten und gelösten Bestandteilen in der wässrigen Phase beeinflusst, z.B. durch Gegenionen, zusätzliche Lipide, Art des zu verkapselnden Wirkstoffs etc. Der Zahlenwert der cmc lässt sich nur experimentell für das betreffende System ermitteln und zwar indirekt durch elektrochemische Verfahren, z.B. Leitfähigkeitsmessungen oder potentiometrische Bestimmung der Gegenionen mit Hilfe einer geeignete Elektrode, durch Messung der Ueberführungszahl, der Oberflächenspannung, Messung von kolligativen Eigenschaften wie Dampfdruckerniedrigung, Gefrierpunktserniedrigung und osmotischer Druck, Messung der Dichte, des Brechungsindex, der Absorption von UV- und IR-Licht, der Solubilisation löslicher und unlöslicher Farbstoffe, der Lichtstreuung, der Fluoreszenzpolarisation und der Viskosität. Diese Eigenschaften erfahren bei Erreichen der cmc eine deutliche Aenderung. So ist die Oberflächenspannung in Abhängigkeit von der Konzentration des ionischen Tensids bis zum Erreichen der cmc durch eine starke Abnahme gekennzeichnet, oberhalb der cmc bleibt die Oeberflächenspannung praktisch konstant. Es wird auf die Ausführung in Stache H., Tensidtaschenbuch, Hanser, 1981, verwiesen, insbesondere S. 26, 3.1. "Methoden zur cmc-Bestimmung" und S. 28, 3.2. "Abhängigkeit der cmc von verschiedenen Parametern". Spezifische cmc-Werte, z.B. für Dodecylpyridiniumbromid, sind in Adderson J.B. and Taylor H., J. Colloid. Sci. 19, 495 (1964) angegeben. Ist die cmc überschritten, kann man durch Verdünnen der wässrigen Phase mit Wasser die cmc wieder unterschreiten. Aus Mizellen bilden sich dann reversibel unilamellare Liposomen.

Wässrige Phasen mit einem pH-Wert höher als ca. 8 werden nach der Dispersion neutralisiert, z.B. auf den physiologischen pH-Wert von 7,2. Die Neutralisation ist notwendig, um eine Zerstörung des Wirkstoffs und/oder der Liposomen unter basischen Bedingungen zu vermeiden und um die physiologische Verträglichkeit der applizierbaren wässrigen Phase mit der Liposomenmischung zu gewährleisten. Man neutralisiert mit einer physiologisch verträglichen Säure oder einer Pufferlösung mit einem pH-Wert von 7 bis 8. Physiologisch verträgliche Säuren sind beispielsweise verdünnte wässrige Mineralsäuren, z.B. verdünnte Salzsäure, Schwefelsäure oder Phosphorsäure, oder organische Säuren, z.B. Niederalkancarbonsäuren, z.B. Essigsäure.

Wässrige Phasen mit kationischen Tensiden der Formel IA können sauer reagieren. Diese neutralisiert man durch Zugabe von verdünnten wässrigen Basen, z.B. verdünnter wässriger Natronlauge oder Kalilauge oder mit einer Pufferlösung vom pH-Wert 7 bis 8.

Man arbeitet zweckmässigerweise bei Raumtemperatur oder auch bei höheren Temperaturen, z.B. bis ca. 60°C, und unter Rühren oder Schütteln. Falls es die Empfindlichkeit des zu verkapselnden Wirkstoffs verlangt, führt man das Verfahren unter Kühlen und gegebenenfalls Inertgasatmosphäre, z.B. Stickstoffatmosphäre,

durch. Die so erhältlichen Liposomen sind in wässriger Phase relativ lange (bis zu mehreren Tagen) beständig. Wässrige Phasen mit erfindungsgemäss herstellbaren unilamelaren Liposomen können nach dem in der Europäischen Patentanmeldung 00 65 292 angegebenen Verfahren lagerungsfähig gemacht werden.

Die Grösse der gebildeten unilamellaren Liposomen ist u.a. von der Struktur der Tenside und der Lipidkomponenten, dem Mischungsverhältnis der Komponent, der Konzentration dieser Komponenten in der wässrigen Phase und von der Menge und Struktur des zu verkapselnden Wirkstoffs abhängig. So kann man beispielsweise durch Erhöhung der Konzentration der Tensidkomponenten wässrige Phasen mit einem hohen Anteil an kleinen oder grossen unilamellaren Liposomen herstellen. Zusätzlich zu KUL entstehen auch grosse unilamellare Liposomen (GUL-Durchmesser bis zu 50,000 Å), Diese schliessen grössere Volumina pro Mol eingesetzter Lipidkomponenten ein und eignen sich zur Verkapselung von voluminösen Materalien, z.B. Viren, Bakterien oder Zellorganellen.

Die Trennung der KUL von GUL erfolgt mittels herkömmlicher Trennmethoden, z.B. Gelfiltration, z.B. mit Sepharose 4B oder Sephacryl als Träger, oder durch Sedimentation der GUL in der Ultrazentrifuge z.B. bei 160,000 x g. Beispielsweise setzen sich nach mehrstündigem, ca. dreistündigem, Zentrifugieren in diesem Schwerefeld GUL ab, während die KUL dispergiert bleiben und dekantiert werden können. Nach mehrmaligem Zentrifugieren erreicht man eine vollständige Trennung der GUL von KUL.

Auch durch Gelfiltration kann man alle in der wässrigen Phase befindlichen Liposomen mit einem Durchmesser grösser als 600 Å, z.B. GUL oder multilamellare Liposomen, sowie nicht verkapselte Wirkstoffe und überschüssige, dispergierte Lipide abtrennen und so eine wässrige Phase mit einer Fraktion KUL von relativ einheitlicher Grösse erhalten.

Nach Abtrennung von grossen unilamellaren (GUL) und multilamellaren Liposomen mit einer der genannten Methoden lässt sich die erfolgte Bildung von kleinen unilamellaren Liposomen und ihr Gehalt in wässriger Phase in an sich bekannter Weise anhand verschiedener physikalischer Messmethoden nachweisen, z.B. mit gefriergebrochenen (freeze fracture) Proben und Dünnschnitten im Elektronenmikroskop oder durch Röntgendiffraktion, durch dynamische Lichtstreuung, durch Massenbestimmung des Filtrats in der analytischen Ultrazentrifuge und vor allem spektroskopisch, z.B. im Kernresonanzspektrum ($^{1}H$, $^{13}C$ und $^{31}P$). So ergeben beispielsweise scharfe Signale mit schmaler Linienbreite im Kernresonanzspektrum einen Hinweis auf erfolgte Bildung von unilamellaren Liposomen mit einem Durchmesser kleiner als ca. 1000 Å. Scharfe Signale bei $\delta$ ca. 0,89 ppm ($-CH_3$), $\delta$ ca. 1,28 ppm ($-CH_2-$) und $\delta$ ca. 3,23 ppm ($-N(CH_3)_3$) sind z B. für verfahrensgemäss erhaltene unilamellare Liposomen mit Phosphatidylcholin als Bestandteil charakteristisch. Im Kernresonanzspektrum sind solche Signale für unilamellare Liposomen typisch und unterscheiden sich von gemischten Mizellen, z.B. aus Phospholipiden, z.B. Lecithin, und Tensiden, z.B. Cetyltrimethylammoniumbromid. Für gemischte Mizellen mit diesen Komponenten ist ein Methylsignal bei $\delta$ ca. 0,89 ppm charakteristisch, welches zu einem Triplett aufgespalten ist und eine wesentlich geringere Linienbreite hat als das Methylsignal (Singlett) (ebenfallsbei $\delta$ ca. 0,89 ppm) das von unilamellaren Liposomen stammt.

Die erfindungsgemäss erhältlichen Liposomen (KUL und GUL) sind geeignete Trägersystem, welche in wässriger Phase zur Solubilisierung von lipophilen Stoffen, z.B. fettlöslichen Farbstoffen, zur Stabilisierung von hydrolyseempfindlichen Stoffen, z.B. Prostaglandinen, zum Einschluss von Schädlingsbekämpfungsmitteln, z.B. zur Veränderung des Wirkungsprofils von Dichlorphos, zum Einschluss von Nahrungsmittelzusätzen, z.B. zwecks Aenderung des Adsorptionsverhaltens von Vitaminen oder Farbstoffen, oder zur Einschleusung von verkapselten Wirkstoffen, Enzymen, Antikörpern, Hormonen, Genen, Viren, Vitaminen oder Zellorganellen in die Zellen einer Zellkultur verwendet werden können.

Wässrige Phasen, welche die erfindungsgemäss erhältlichen Liposome mit verkapselten Wirkstoffen enthalten, sind Verbreichungssysteme, welche sich, gegebenenfalls nach Konzentrierung oder Isolierung der Liposomen, z.B. durch Ultrazentrifugieren, zu therapeutischen Zwecken für die orale (p.o.), parenterale (i.v., i.m. oder i.p.) oder topikale Verabreichung eignen.

Bei oraler Verabreichung können Verabreichungssysteme auf Liposomenbasis einen Wirkstoff, beispielsweise Insulin, das im Verdauungstrakt unbeständig ist, schützen oder seine Resorption verbessern. Für die orale Verabreichung kann die Liposomen-haltige wässrige Phase mit pharmazeutisch unbedenklichen Verdünnungsmiteln oder Trägern oder mit üblichen Zusätzen, z.B. Farbstoffen oder Geschmacksstoffen, vermischt und als Sirup oder in Form von Kapseln verabreicht werden.

Bei parenteraler Verabreichung können Verabreichungssysteme auf Liposomenbasis beispielsweise die Verweilzeit z.B. von Desferrioxamin, siehe Guilmette R.A. et al., Life Sci. 22 (4) 313-320, 1978, oder Gentamycin, siehe Scheld W.M. et al., Clin.Res. 26, No. 1, 59 A, 1978, in einem Organismus verlängern. Ebenso wird die Verweilzeit von verkapselten Chelatbildern, z.B. EDTA (Aethylendiamintetraessigsäure), in Organismen verlängert, so dass man durch Chelatbildung Schwermetalle besonders aus Leber, Milz oder Nieren entfernen kann, siehe Rahmann et al, Science, Vol 180,300-302, 1973, und J. Lab.Clin. Med. 640-647, 1974. Mit Verabreichungssystemen auf Liposomenbasis kann man Wirkstoffe im Myokard anreichern, siehe Landesmann et al, Science Vol. 198, 737-738, 1977. Antiinflammatorisch wirkende Stoffe, z.B. Cortisol, siehe Nature 271, No. 5643, 372-73, 1978, oder Proteaseinhibitoren, siehe Anal. Biochem. 89, No.2, 400-07, 1978, kann man in der Gelenkflüssigkeit und Cytostatika in Tumorgewebe, siehe Uebersichtsartikel von Kaye St. B., Cancer Treatment Reviews 8, 27-50, 1981, und die vielen darin zitierten Literaturstellen, anreichern. Manche Chemotherapeutika in der Krebstherapie sind weniger toxisch und besser verträglich, wenn

sie in Liposomen verkapselt verabreicht werden, z.B. liposomverkapseltes Actinomycin D, siehe Rahman et al, Proceedings of the Society for Experimental Biology and Medicine 146, 1173-1176, 1974, Methotrexat, siehe Lesermann L.D. et al. Proc. Natl. Acad. Sci. 77, No. 7, 4089-93, 1980, Vinblastin, Daunomycin oder Cytosin-Arabinosid, siehe Mühlensiepen et al., Cancer Res. 41, Nr. 5, 1602-07, 1981. Liposomen können zur Einschleusung von Wirkstoffen, z.B. Enzymen, Peptidhormonen, Genen oder Viren in das Cytoplasma von Zellen in lebenden Organismen, z.B. zur Einschleusung von Asparaginase, siehe Uebersichtsartikel von Finkelstein M. und Weissmann, G., J. Lipid Research, Vol. 19, 1978, 289-303, von Amyloglucosidase, siehe Gregoriadis G. und Ryman B.E., Eur. J.Biochem. 24 (1972), 485-491, oder Neurominidase, siehe Gregoriadis et al., Biochem. J. (1974) 140, 232-330, zur Verankerung spezifischer Erkennungsmoleküle, z.B. monoklonaler Antikörper, zwecks zielgerichteter Einschleusung in definierte Zielzellen, siehe Leserman et al., Nature 293 (5829), 226-228, 1981, zur Immunstimulation als Adjuvans bei Impfungen, z.B. gegen Leishmaniasen, siehe New R.R.C. et al. Nature 272 (5648) 55-56, 1978, oder zur induzierten Freisetzung von Wirkstoffen durch Signale wie Temperaturerhöhungen, z.B. in entzündetem Gewebe, oder pH-Wert Aenderungen verwendet werden. Für die parenterale Verabreichung können die konzentrierten oder isolierten Liposomen in einer geeigneten Trägerflüssigkeit, beispielsweise in sterilem destilliertem Wasser oder in physiologischer Kochsalzlösung, suspendiert werden.

Herstellung der homogenen Schicht der Lipidkomponenten

Die Herstellung der homogenen Schicht der Lipidkomponenten kann in an sich bekannter Weise erfolgen. Beispielsweise löst man zunächst das Tensid der Formel IA, z.B. Cetylpyridiniumchlorid und das Lipid, z.B. Ei-Lecithin, gegebenenfalls unter Zumischung eines lipophilen Wirkstoffs, z.B. Proteins, das bei der Bildung der Liposomen in der Lipidschicht eingeschlossen wird, in einem organischen Lösungsmittel auf. Durch Entfernen des organischen Lösungsmittels, am

zweckmässigsten im Vakuum oder durch Abblasen mit Inertgas, z.B. Stickstoff, stellt man eine aus einem Film bestehende homogene Schicht der Lipidkomponenten her.

Die Auswahl des betreffenden Lösungsmittels ist von der Löslichkeit der betreffenden Lipidkomponenten darin abhängig. Geeignete Lösungsmittel sind beispielsweise unsubstituierte oder substituierte, z.B. halogenierte, aliphatische, cycloaliphatische, aromatische oder aromatisch-aliphatische Kohlenwasserstoffe, z.B. Benzol, Toluol, Methylenchlorid oder Chloroform, Alkohole, z.B. Methanol oder Aethanol, Niederalkancarbonsäureester, z.B. Essigsäureäthylester, Aether, z.B. Diäthyläther, Dioxan oder Tetrahydrofuran, oder Mischungen dieser Lösungsmittel.

Eine homogene Mischung kann man auf die in der DE-A 28 18 655 beschriebene Weise durch lyophilisieren aus organischer Lösung herstellen. Man erhält die homogene Schicht in Form eines Schaums.

Die in der Beschreibung erwähnten ionischen Tenside, z.B. die kationischen Tenside der Formel IA und die anionischen Tenside der Formel IB sind bekannt. Die Herstellung dieser Tenside ist in dem Standardwerk "Cationic surfactants" von Eric Jungermann, Dekker, New York 1970, beschrieben. Eine Uebersicht über sämtliche im Handel befindlichen anionischen und kationischen Tenside, sowie die Warenzeichen, unter denen diese Tenside von den Herstellerfirmen vertrieben werden, gilt das jährlich neu erscheinende Handbuch "Mc Cutcheon's, Emulsifiers & Detergents", Manufacturing Confectioner Publishing Co. Die Tenside der Formeln I B und I C sind bekannt oder können falls sie neu sind, in an sich bekannter Weise nach den im Standardwerk von Knight C.G., Liposomes, Elsevier 1981, Kapitel 3, angegebenen Vorschriften hergestellt werden. Die weiter vorn genannten Lipide sind bekannt und grösstenteils handelsüblich.

Die folgenden Beispiele veranschaulichen die Erfindung, ohne sie zu beschränken. Temperaturen sind in Grad Celsius und chemische Verschiebungen (δ) im NMR-Spektrum in ppm angegeben.

Beispiel 1: Man löst 10 mg Ei-Lecithin und 0,05 mg Cetyltrimethylammoniumbromid in 2 ml einer Chloroform/ Methanol-Mischung (2:1) und dampft diese Lösung im Vakuum im Rotationsverdampfer ein. Zur Bildung von unilamellaren Liposomen dispergiert man bei Raumtemperatur den filmartigen Rückstand in 1 ml Wasser durch 5-10 Minuten langes Schütteln. Man erhält eine leicht opaleszierende, wässrige Phase.

Die erfolgte Bildung von kleinen unilamellaren Liposomen ist im NMR-Spektrum durch die Signale $\delta = 1{,}28$ (Methylen). $\delta = 0{,}86$ (Methyl) und $\delta = 3{,}25$ ($-N(CH_3)_3$) erkennbar.

Die gebildeten unilamellaren Liposomen können im Elektronenmikroskop sichtbar gemacht werden. Die Liposomendispersion wird zunächst der üblichen Gefrierbruchmethode (freeze-fracture) unterzogen. Es liegen hauptsächlich zwei "Populationen" von unilamellaren Liposomen vor, die sich durch ihre durchschnittliche Grösse unterscheiden:
1. Kleine unilamellare Liposomen (KUL) mit einem Durchmesser von ca. 200-600 Å und
2. Grosse unilamellare Liposomen (GUL) mit einem Durchmesser von ca. 1,000 - 10,000 Å.

Beispiel 2: Analog Beispiel 1 löst man pro Versuch 10 mg Ei-Lecithin und eine steigende Menge Cetyltrimethylammoniumbromid (CTAB, siehe Tabelle) in je 2 ml einer Chloroform/Methanol-(2:1)-Mischung, dampft ein und dispergiert in Wasser. Man erhält eine opaleszierende wässrige Phase, welche aus kleinen (KUL) und grossen (GUL) unilamellaren Liposomen besteht.

Tabelle 1:

| Versuch Nr. | Konzentration CTAB [g/l] | Ausbeute KUL [%] |
|---|---|---|
| 1 | 0,1 | 10 |
| 2 | 0,2 | 10 |
| 3 | 0,5 | 10 |
| 4 | 1,0 | 10 |
| 5 | 2,0 | 12 |
| 6 | 5,0 | 14 |
| 7 | 7,0 | 20 |
| 8 | 10,0 | 40 |
| 9 | 15,0 | 70 |

Beispiel 3: Man löst pro Versuch 10 mg Ei-Lecithin und eine steigende Menge Cetylpyridiniumchlorid (CPC, siehe Tabelle 2) oder Benzalkoniumchlorid (BAC, siehe Tabelle 3) in je 2 ml einer Chloroform/Methanol - (2:1) - Mischung, dampft diese Lösung im Vakuum ein und dispergiert in 1 ml Wasser durch fünf bis zehn Minuten langes Schütteln. Man erhält eine opaleszierende, wässrige Phase, welche aus kleinen (KUL) und grossen (GUL) unilamellaren Liposomen besteht.

Tabelle 2:

| Versuch Nr. | Konzentration CPC [g/l] | Ausbeute KUL [%] |
|---|---|---|
| 1 | 1,0 | 10 |
| 2 | 1,5 | 15 |
| 3 | 2,0 | 20 |
| 4 | 2,5 | 20 |
| 5 | 3,0 | 25 |
| 6 | 3,5 | 30 |

Tabelle 3:

| Versuch Nr. | Konzentration BAC [g/l] | Ausbeute KUL [%] |
|---|---|---|
| 1 | 0,5 | 2 |
| 2 | 1,0 | 5 |
| 3 | 2,0 | 5 |
| 4 | 3,0 | 10 |
| 5 | 5,0 | 15 |
| 6 | 10,0 | 60 |

Beispiel 4: Man löst pro Versuch 10 mg Ei-Lecithin und eine steigende Menge an Texapon N 25® (Natriumlauryläthersulfat, siehe Tabelle 4), Octadecylphospho-D-mannit (OPM, siehe Tabelle 5) oder Natriumdodecylsulfat (SDS, Tabelle 6) in je 2 ml einer Chloroform/Metanol-(2:1)-Mischung, dampft diese Lösung im Vakuum ein und dispergiert in 1 ml Wasser durch fünf bis zehn Minuten langes Schütteln. Man erhält eine opaleszierende, wässrige Phase, welche aus kleinen (KUL) und grossen (GUL) unilamellaren Liposomen besteht.

Tabelle 4:

| Versuch Nr. | Konzentration Texapon N 25 [g/l] | Ausbeute KUL [%] |
|---|---|---|
| 1 | 1,0 | 2 |
| 2 | 2,0 | 5 |
| 3 | 3,0 | 5 |
| 4 | 4,0 | 10 |

Tabelle 5:

| Versuch Nr. | Konzentration OPM [g/l] | Ausbeute KUL [%] |
|---|---|---|
| 1 | 1,0 | 10 |
| 2 | 2,0 | 15 |
| 3 | 3,0 | 20 |

Tabelle 6:

| Versuch Nr. | Konzentration SDS [g/l] | Ausbeute KUL [%] |
|---|---|---|
| 1 | 1,0 | 5 |
| 2 | 2,0 | 8 |
| 3 | 3,0 | 10 |
| 4 | 4,0 | 12 |
| 5 | 5,0 | 15 |
| 6 | 6,0 | 15 |
| 7 | 7,0 | 20 |
| 8 | 8,0 | 30 |
| 9 | 9,0 | 35 |

Beispiel 5: Man löst jeweils eine Gesamtmenge von 10 mg enthaltend den in den Tabellen 1-3 angegebenen Anteil an Natrium-2,2-dimethyl-3-palmitoyloxypropylhydrogenphosphat (Tabelle 7), Natrium-1-palmitoyllysophosphatidylglycerin (Tabelle 8) und Natrium-1-palmitoyllysophosphatidylserin (Tabelle 9) und die entsprechende Menge Ei-Lecithin (Lipid) in 1 ml einer Chloroform/Methanol Mischung (2:1) und dampft diese Lösung im Rotationsverdampfer ein. Anschliessend dispergiert man den filmartigen Rückstand in 1 ml destilliertem Wasser und neutralisiert durch Zugabe von 0,1 N Natriumhydroxid-Lösung. Man erhält eine opaleszierende wässrige Phase.

Tabelle 7:

| Versuch Nr. | Konzentration Tensid [g/l] | Ausbeute KUL [%] |
|---|---|---|
| 1 | 0.5 | 7 |
| 2 | 1,0 | 13 |
| 3 | 1,5 | 19 |
| 4 | 2,0 | 23 |
| 5 | 2,5 | 26 |
| 6 | 3,0 | 30 |
| 7 | 4,0 | 37 |
| 8 | 5,0 | 60 |
| 9 | 6,0 | 83 |
| 10 | 7,0 | 90 |
| 11 | 8,0 | 95 |
| 12 | 9,0 | 100 |
| 13 | 9,5 | 100 |

Bei einen Anteil von mehr als 60 % Tensid enthalten die KUL-Fraktionen ausserdem kleine Mizellen aus Lipid und Tensid.

Tabelle 8:

| Versuch Nr. | Konzentration Tensid [g/l] | Ausbeute KUL [%] |
|---|---|---|
| 1 | 1,0 | 6 |
| 2 | 1,5 | 10 |
| 3 | 2,0 | 15 |
| 4 | 2,5 | 17 |
| 5 | 3,0 | 20 |
| 6 | 3,5 | 25 |
| 7 | 4,0 | 27 |
| 8 | 4,5 | 30 |
| 9 | 5,0 | 33 |
| 10 | 6,0 | 40 |

Tabelle 9:

| Versuch Nr. | Konzentration Tensid [g/l] | Ausbeute KUL [%] |
|---|---|---|
| 1 | 1 | 5 |
| 2 | 2 | 8 |
| 3 | 3 | 13 |
| 4 | 4 | 18 |
| 5 | 5 | 20 |
| 6 | 6 | 25 |

Beispiel 6: Man löst 3 mg eines in der Tabelle 10 genannten Tensids und 7 mg Ei-Lecithin (Lipid) in 1 ml einer Chloroform/Methanol Mischung (2:1) und dampft diese Lösung ein. Der filmartige Rückstand wird in 1 ml Wasser dispergiert. Anschliessend neutralisiert man durch Zugabe von 0,1 N Natriumhydroxidlösung. Man erhält eine opaleszierende, wässrige Phase.

Tabelle 10:

| Tensid | Ausbeute [% KUL] |
|---|---|
| 2-Hydroxyäthyl-3-palmitoyloxypropylphosphat | 20 |
| 2,2-Dimethyl-3-palmitoyloxypropylhydrogenphosphat | 50 |
| 3-Cetyloxypropyl-2-hydroxyäthylphosphat | 29 |
| 2-Bromäthyl-cetylphosphat | 30 |
| n-Eicosyl-2,3-(2,2-propylen)-dioxypropylphosphat | 18 |
| 3-Stearyloxypropylhydrogenphosphat | 8 |
| 2,3-Dihydroxypropyl-myristylphosphat | 34 |
| 3-Cetyloxypropylhydrogenphosphat | 19 |
| 2,3-Dihydroxypropyl-n-eicosylphosphat | 8 |

| | |
|---|---|
| Cetyl-2,3-dihydroxypropylphosphat | 25 |
| Methyl-3-stearoyloxypropylphosphat | 45 |

Beispiel 7: Man löst 20 mg (0,026 mMol) Sojalecithin, 1 mg (0,76 µMol) N-Acetylmuramyl-L-alanyl-2-(1',2'-dipalmitoyl-sn-glycero-3'-phosphoryl)-äthylamid und 5 mg n-Hexadecylpyridiniumchlorid in 2 ml einer Chloroform/Methanol-Mischung (2:1) und dampft diese Lösung im Rotationsverdampfer ein. Der filmartige Rückstand wird in 3 ml destilliertem Wasser fünf Minuten lang geschüttelt. Man erhält eine opaleszierende, wässrige Phase. Man puffert anschliessend die wässrige Dispersion durch Zugabe von 0,2 ml eines zehnfachen Konzentrats von Phosphat-gepufferter,isotonischer Kochsalzlösung (PBS für Injektionszwecke) auf den pH-Wert von 7,4 ab.

Beispiel 8: Man löst 30 mg (0,04 mMol) Sojalecithin, 2 mg (0,004 mMol) Flumethason-21-pivalat und 8 mg (0,002 mMol) n-Hexadecylpyridiniumchlorid in 3 ml einer Chloroform/Methanol Mischung (2:1) und dampft diese Lösung im Rotationsverdapfer ein. Der filmartige Rückstand wird in 3 ml destilliertem Wasser fünf Minuten lang geschüttelt. Man erhält eine opaleszierende, wässrige Phase. Anschliessend puffert man wie im Beispiel 7 beschrieben auf den pH-Wert von 7,4 ab.

Beispiel 9: Man löst 30 mg (0,040 mMol) Sojalecithin und 15 mg (0,042 mMol) Lanette E ® (Natrium-stearyl- oder palmitylsulfat) in 8 ml einer tert-Butanol/Methanol Mischung (4:1) bei 70° und dampft diese Lösung im Vakuum ein. Der filmartige Rückstand wird mit 3 ml destilliertem Wasser fünf Minuten lang geschüttelt. Man erhält eine opaleszierende, wässrige Phase, welche man wie im Beispiel 7 beschrieben auf den pH-Wert von 7,4 abpuffert.

Beispiel 10: Man löst 20 mg (0,026 mMol) Sojalecithin, 1 mg

(0,76 Mol) N-Acetylmuramyl-L-alanyl-2-(1',2'-dipalmitoyl-sn-glycero-3'-phosphory 1)-äthylamid und 10 mg (0,028 mMol) Lanette E ® in 6 ml einer tert-Butanol/Methanol Mischung (4:1) bei 70° und dampft diese Lösung im Rotationsverdampfer ein. Der filmartige Rückstand wird in 2 ml destilliertem Wasser 5 Minuten lang geschüttelt. Man erhält eine opaleszierende, wässrige Phase.

Diese wird in eine gerührte Ultrafiltrationszelle (Amicon ®), eingefüllt, die anstelle des Ultrafilters mit einem geradporigen Filter aus Polycarbonat (Nucleopore ®) mit einem Porendurchmesser von 0,1 µm versehen ist und partikelfrei gewaschen wurde, und unter geringem Ueberdruck und stetiger Zufuhr von sterilfiltrierter Pufferlösung nach Dulbecco (pH 7,4 ohne Ca und Mg) so filtriert, dass das Volumen in der Zelle nicht unter 30 ml sinkt. Nach Durchtritt von 0,3 l Filtrat sind alle KUL abgetrennt und die überstehende Dispersion an GUL kann ampulliert und für Behandlungsversuche eingesetzt werden.

Beispiel 11: Man löst 30 mg (0,04 mMol) Sojalecithin, 4 mg (0,081 mMol) Flumethason-21-pivalat und 10 mg (0,028 mMol) Lanette E ® in 6 ml einer tert-Butanol/Methanol Mischung (4:1) bei ca. 70° und dampft diese Lösung im Rotationsverdampfer ein. Der filmartige Rückstand wird in 3 ml destilliertem Wasser geschüttelt. Man erhält eine opaleszierende, wässrige Phase. Nach Einfüllen in eine gerührte Filterzelle (Totalvolumen 100 ml) gemäss Beispiel 10 wird unter Zugabe von sterilem, partikelfrei filtriertem Wasser so lange filtriert, bis 500 ml Filtrat gesammelt sind. Dieses Filtrat wird in eine gerührte Filterzelle, die mit einem Ultrafilter, z.B. Amicon U 10 ®, bestückt ist, kontinuierlich eingespeist und auf ein Volumen von 30 ml konzentriert. Die konzentrierte Dispersion enthält kleine, unilamellare Liposomen und kann nach Zugabe eines Konzentrats von Phosphatpuffer nach Dulbecco (pH 7,4 ohne Ca und Mg) ampulliert und für Behandlungsversuche eingesetzt werden.

Ansprüche

1. Verfahren zur Herstellung von unilamellaren Liposomen in wässriger Phase, dadurch gekennzeichnet, dass man eine homogene Mischung eines ionischen Tensids und eines Lipids in wässriger Phase bei einer Konzentration niedriger als die kritische Mizellbildungskonzentration (cmc-critical micelle concentration) des Tensids in der betreffenden Phase dispergiert und, wenn notwendig, die erhältliche wässrige Phase neutralisiert und, wenn erwünscht, die erhältlichen unilamellaren Liposomen anreichert und/oder abtrennt.

2. Verfahren nach Anspruch 1, dadurch gekennzeichnet, dass man eine homogene Mischung eines anionischen oder kationischen Tensids dispergiert.

3. Verfahren nach Anspruch 2, dadurch gekennzeichnet, dass man eine homogene Mischung eines kationischen Tensids der Formel:

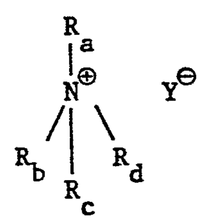

(I A),

worin $R_a$ einen gegebenenfalls substituierten Kohlenwasserstoffrest, $R_b$ Niederalkyl, Phenylniederalkyl oder Hydroxy, $R_c$ und $R_d$ Niederalkyl oder $R_b$ und $R_c$ zusammen mit dem Stickstoffatom einen gegebenenfalls an einem Kohlenstoffatom substituierten, aliphatischen Heterocyclus und $R_d$ Niederalkyl oder $R_b$, $R_c$ und $R_d$ zusammen mit dem Stickstoffatom einen gegebenenfalls an einem Kohlenstoffatom substituierten, aromatischen Heterocyclus und $Y^{\ominus}$ ein Anion darstellen und eines Lipids dispergiert.

4. Verfahren nach Anspruch 3, dadurch gekennzeichnet, dass man eine homogene Mischung von N-Benzyl N,N-dimethyl-N-2-[2-(4-(1,1,3,3-tetramethylbutyl)-phenoxy)-äthoxy]-äthylammoniochlorid, N-Benzyl-N,N-dimethyl-N-2-[2-(3-methyl-4-(1,1,3,3-tetramethylbutyl)-phenoxy)-äthoxy]-äthylammoniochlorid (Methylbenzethoniumchlorid), n-Dodecyltrimethylammoniochlorid oder -bromid, Trimethyl-n-tetradecylammoniochlorid oder -bromid, n-Hexadecyltrimethylammoniochlorid oder -bromid (Cetyltrimethylammoniumchlorid oder -bromid), Trimethyl-n-octadecylammoniochlorid oder -bromid, Aethyl-n-dodecyldimethylammoniochlorid oder -bromid, Aethyldimethyl-n-tetradecylammoniochlorid oder -bromid, Aethyl-n-hexadecyldimethylammoniochlorid oder -bromid, Aethyldimethyl-n-octadecylammoniochlorid oder -bromid, n-Alkyl-benzyldimethylammoniochlorid oder -bromid (Benzalkoniumchlorid oder -bromid), z.B. Benzyl-n-dodecyldimethylammoniochlorid oder bromid, Benzyldimethyl-n-tetradecylammoniochlorid oder -bromid, Benzyl-n-hexadecyldimethylammoniochlorid oder -bromid oder Benzyldimethyl-n-octadecylammoniochlorid oder -bromid, N-(n-Decyl)-pyridiniochlorid oder -bromid, N-(n-Dodecyl)-pyridiniochlorid oder -bromid, N-(n-Tetradeyl)-pyridiniochlorid oder -bromid, N-(n-Hexadecyl)-pyridiniochlorid oder -bromid (Cetylpyridiniumchlorid) oder N-(n-Octadecyl)-pyridiniochlorid oder -bromid oder eine Mischung von diesen Tensiden und eines Lipids dispergiert.

5. Verfahren nach Anspruch 2, dadurch gekennzeichnet, dass man eine homogene Mischung eines anionischen Tensids der Formel:

a) eine Verbindung der Formel:

$$[R_a-(O-A)_m-B]^{\ominus}Z^{\oplus} \qquad \text{(I B)}$$

worin $R_a$ einen gegebenenfalls substituierten Kohlenwasserstoffrest, A Niederalkylen, m null (direkte Bindung) oder eins, B die Sulfonat- oder Sulfatgruppe und $Z^{\oplus}$ ein einwertiges Kation darstellen, oder

b) eine Verbindung der Formel:

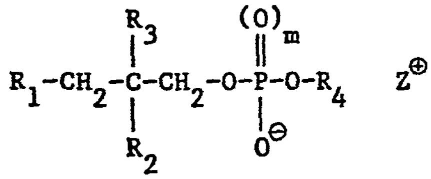

(I C),

worin m null oder eins ist, einer der Reste $R_1$ und $R_2$ Wasserstoff, Hydroxy, Niederalkyl mit 1-4 C-Atomen und der andere Rest Alkyl, Alkenyl, Alkoxy, Alkenyloxy oder Acyloxy mit je 10-20 C-Atomen, $R_3$ Wasserstoff oder Niederalkyl mit 1-4 C-Atomen und $R_4$ gegebenenfalls substituiertes Niederalkyl mit 1-7 C-Atomen, einen Kohlehydratrest mit 5-12 C-Atomen oder, wenn beide Reste $R_1$ und $R_2$ Wasserstoff oder Hydroxy bedeuten, einen Steroidrest bedeuten, und $Z^{\oplus}$ ein einwertiges Kation bedeutet, oder

c) eine Verbindung der Formel

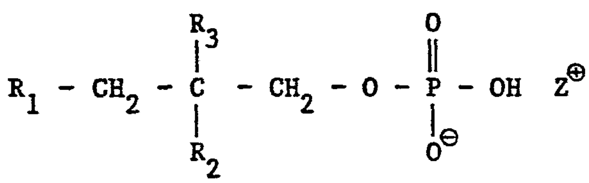

(I D),

worin $R_1$, $R_2$, $R_3$ und $Z^{\oplus}$ die unter Formel (I C) genannten Bedeutungen haben, und eines Lipids dispergiert.

6. Verfahren nach Anspruch 5, dadurch gekennzeichnet, dass man eine homogene Mischung enthaltend ein Alkalimetallalkylsulfat (m = o), z.B. Natrium oder Kalium-n-dodecyl (lauryl)-sulfat, -n-tetradecyl (myristyl)-sulfat, -n-hexadecyl (cetyl)-sulfat oder -n-octadecyl (stearyl)-sulfat, ein Alkalimetallalkyläthersulfat (m = 1), z.B. Natrium- oder Kalium-n-dodecyloxyäthylsulfat, -n-tetradecyloxyäthylsulfat, -n-hexadecyloxyäthylsulfat oder -n-octadecyloxyäthylsulfat oder , ein Alkalimetallalkansulfonat, z.B. Natrium- oder Kalium-n-dodecansulfonat, n-tetradecansulfonat, n-hexadecansulfonat oder -n-dodecansulfonat, das Natrium- oder Kaliumsalz des Lysophosphatidylserins, z.B. das Natrium- oder Kaliumsalz des Lysophosphatidylserins aus dem Rinderhirn oder das Natrium- oder

Kaliumsalz eines synthetischen Lysophosphatidylserins, z.B. Natrium- oder Kalium-1-myristoyl- oder -1-palmitoyllysophosphatidylserin, oder das Natrium- oder Kaliumsalz des Lysophosphatidylglycerins, oder Natrium- oder Kaliumsalz einer natürlichen Phosphatidsäure, z.B. Ei-Phosphatidsäure, das Natrium- oder Kaliumsalz einer natürlichen Lysophosphatidsäure, z.B. Ei-Lysophosphatidsäure, das Natrium- oder Kaliumsalz einer synthetischen Lysophosphatidsäure, z.B. 1-Lauroyl-, 1-Myristoyl- oder 1-Palmitoyllysophosphatidsäure, oder einer Mischung von diesen Tensiden und eines Lipids dispergiert.

7. Verfahren nach einem der Ansprüche 1-6, dadurch gekennzeichnet, dass man als Lipid eine Verbindung der Formel

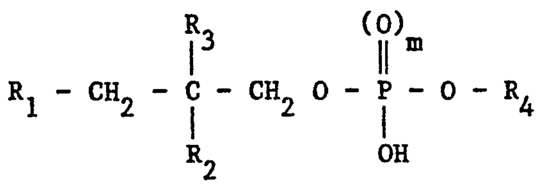

(I C'),

worin m, $R_1$, $R_2$, $R_3$ und $R_4$ die unter Formel I C genannten Bedeutungen haben und $R_4$ ausserdem durch Triniederalkylammonio oderAmino substituiertes Niederalkyl ist, dispergiert.

8. Verfahren nach Anspruch 7, dadurch gekennzeichnet, dass man als Lipid ein natürliches Lecithin, z.B. Ei-Lecithin oder Lecithin aus Sojabohnen ($R_4$ = 2-Trimethylammonioäthyl), ein natürliches Kephalin, z.B. Ei-Kephalin oder Kephalin aus Sojabohnen ($R_4$ = 2-Aminoäthyl), ein synthetisches Lecithin ($R_4$ = 2-Trimethylammonioäthyl) oder ein synthetisches Kephalin ($R_4$ = 2-Aminoäthyl) der Formel (I C') bevorzugt, worin $R_1$ und $R_2$ identische Acyloxyreste, z.B. Lauroyloxy, Oleoyloxy, Linoyloxy, Linoleoyloxy oder Arachinoyloxy bedeuten, z.B. Dilauroyl-, Dimyristoyl-, Dipalmitoyl-, Distearàroyl-, Diarachinoyl-, Dioleoyl-, Dilinoyl-, Dilinoleoyl-, oder Diarachinoyllecithin oder -kephalin, $R_1$, und $R_2$ verschiedene Acyloxyreste, z.B. $R_1$ Palmitoyloxy und $R_2$ Oleoyloxy, z.B. 1-Palmitoyl-2-oleoyl-lecithin oder -kephalin, $R_1$ und $R_2$ identische Alkoxyreste, z.B. Tetradecyloxy oder Hexadecyloxy, z.B. Ditetradecyl- oder Dihexadecyllecithin oder

-kephalin, $R_1$ Alkenyl und $R_2$ Acyloxy, z.B. ein Plasmalogen ($R_4$ = Trimethylammonioäthyl), oder $R_1$ Acyloxy, z.B. Myristoyloxy oder Palmitoyloxy, und $R_2$ Hydroxy, z.B. natürliches oder synthetisches Lysolecithin oder Lysokephalin, z.B. 1-Myristoyl- oder 1-Palmitoyllysolecithin oder -kephalin, und $R_3$ Wasserstoff darstellen, dispergiert.

9. Verfahren nach einem der Ansprüche 1-8, dadurch gekennzeichnet, dass man eine homogene Mischung aus einem Tensid der Formel I A, insbesondere N,N-Dimethyl-N-2-[2-(4-(1,1,3,3-tetramethylbutyl)-phenoxy)-äthoxy]-äthylammoniochlorid, N-Benzyl-N,N-dimethyl-N-2-[2-(3-methyl-4-(1,1,3,3-tetramethylbutyl)-phenoxy)-äthoxy]-äthylammoniochlorid (Methylbenzethoniumchlorid), n-Dodecyltrimethylammoniochlorid oder -bromid, Trimethyl-n-tetradecylammoniochlorid oder-bromid, n-Hexadecyltrimethylammoniochlorid oder -bromid (Cetyltrimethylammoniumchlorid oder -bromid), Trimethyl-n-octadecylammoniochlorid oder -bromid, Aethyl-n-dodecyldimethylammoniochlorid oder -bromid, Aethyldimethyl-n-tetradecylammoniochlorid oder -bromid, Aethyl-n-hexadecyldimethylammoniochlorid oder -bromid, Aethyldimethyl-n-octadecylammoniochlorid oder -bromid, n-Alkylbenzyldimethylammoniochlorid oder -bromid (Benzalkoniumchlorid oder -bromid), z.B. Benzyl-n-dodecyldimethylammoniochlorid oder -bromid, Benzyldimethl-n-tetradecylammoniochlorid oder -bromid, Benzyl-n-hexadecyldimethylammoniochlorid oder -bromid oder Benzyldimethyl-n-octadecylammoniochlorid oder -bromid, N-(n-Decyl)-pyridiniochlorid oder -bromid, N-(n-Dodecyl)-pyridiniochlorid oder -bromid, N-(n-Tetradecyl)-pyridiniochlorid oder -bromid, N-(n-Hexadecyl)-pyridiniochlorid oder -bromid (Cetylpyridiniumchlorid) oder N-(n-Octadecyl)-pyridiniochlorid oder -bromid oder einem anionischen Tensid der Formel IB, insbesondere Natrium oder Kalium-n-dodecyl (lauryl)-sulfat, -n-tetradecyl (myristyl)-sulfat, -n-hexadecyl (cetyl)-sulfat oder -n-octadecyl (stearyl)-sulfat, Natrium- oder Kalium-n-dodecyloxyäthylsulfat, -n-tetradecyloxyäthylsulfat, -n-hexadecyloxyäthylsulfat oder -n-octadecyloxyäthylsulfat, oder einem anionischen Tensid der Formel I C, insbesondere Natrium- oder Kalium-2,2-dimethyl-3-palmitoyloxypropylhydrogenphosphat, Natrium-

oder Kalium-1-palmitoyllysophosphatidylglycerin, Natrium- oder Kalium-1-palmitoyllysophosphatidylserin, und einem Lipid der Formel I C', worin $R_1$ und $R_2$ Acyloxy, z.B. Lauroyloxy, Myristoyloxy, Palmitoyloxy oder Stearoyloxy, $R_3$ Wasserstoff und $R_4$ 2-Trimethylammonioäthyl, z.B. ein natürliches Kephalin, z.B. Ei-kephalin-oder Kephalin aus Sojabohnen, oder 2-Aminoäthyl, z.B. ein natürliches Lecitin, z.B. Ei-Lecithin oder Lecithin aus Sojabohnen, dispergiert.

10. Verfahren nach einem der Ansprüche 1-9, dadurch gekennzeichnet, dass man eine homogene Mischung eines Tensids und eines Lipids gemäss Anspruch 9 und eines pharmazeutischen Wirkstoffs dispergerit.

11. Verfahren nach einem der Ansprüche 1-10, dadurch gekennzeichnet, dass man eine homogene Mischung aus einem anionischen Tensid der Formel I B, Ei-Lecithin und einem Muramylpeptid dispergiert.

12. Verfahren nach einem der Ansprüche 1-10, dadurch gekennzeichnet, dass man eine homogene Mischung aus einem kationischen Tensid der Formel I A, Sojalecithin und einem Muramylpeptid dispergiert.

13. Verfahren nach Anspruch 12, dadurch gekennzeichnet, dass man eine homogene Mischung aus n-Hexadecylpyridiniumchlorid, Sojalecithin und N-Acetylmuramyl-L-alanyl-2-(1',2'-dipalmitoyl-sn-glycero-3'-phosphoryl)-äthylamid dispergiert.

16. Verabreichungssystem auf Liposomenbasis für verkapseltes N-Acetylmuramyl-L-alanyl-D-isoglutamyl-L-alanyl-2-(1',2'-dipalmitoyl-sn-glycero-3'-phosphoro)-äthylamid, hergestellt nach den Verfahren gemäss Patentanspruch 1.

17. Pharmazeutische Zusammensetzung enthaltend ein Verabreichungssystem auf Liposomenbasis für verkapselte Wirkstoffe gemäss Anspruch 15, vermischt mit pharmazeutisch verträglichen Zusatzstoffen.

18. Verabreichungssystem gemäss Anspruch 15 zur Anwendung bei der Behandlung des menschlichen oder tierischen Körpers.

19. Pharmazeutische Zusammensetzung gemäss Anspruch 15 zur Anwendung bei der Behandlung des menschlichen oder tierischen Körpers.

20. Die Methode der Behandlung von Erkrankungen des menschlichen oder tierischen Körpers mit Verabreichungssystemen gemäss Anspruch 15.

FO 7.4 RS/eg*